Europäisches Patentamt

European Patent Office

Office européen des brevets

(19)

(11) Numéro de publication : **0 378 549 B1**

# FASCICULE DE BREVET EUROPEEN

(12)

(45) Date de publication du fascicule du brevet :
**15.07.92 Bulletin 92/29**

(21) Numéro de dépôt : **88907020.7**

(22) Date de dépôt : **29.07.88**

(86) Numéro de dépôt international :
**PCT/FR88/00393**

(87) Numéro de publication internationale :
**WO 89/00858 09.02.89 Gazette 89/04**

(51) Int. Cl.[5] : **A61K 35/64,** A61K 33/18,
A61K 33/42, A61K 35/78,
// (A61K35/78, 35:64, 33:42,
33:18, 33:00, 31:05, 31:045)

(54) **COMPOSITION PHARMACEUTIQUE DESTINEE A LA PREVENTION ET AU TRAITEMENT DES MALADIES BACTERIENNES OU VIRALES.**

(30) Priorité : **31.07.87 FR 8710912**

(43) Date de publication de la demande :
**25.07.90 Bulletin 90/30**

(45) Mention de la délivrance du brevet :
**15.07.92 Bulletin 92/29**

(84) Etats contractants désignés :
**AT BE CH DE FR GB IT LI LU NL SE**

(56) Documents cités :
**Rote Liste, 1986, Editio Cantor, (Aulen-
dorf/Württ.) see No. 35 090 "Nephrolithol"**

(56) Documents cités :
**Rote Liste, 1979, Editio Canto (Aulen-
dorf/Württ.) see No. 81 099D "Regenaplex-Nr.
86a", No. 81 189D "Regenaplex-Nr. 50c",
No.81 162D "Regenaplex-Nr. 51a".
Rote Liste, 1961, Editio Cantor (Aulen-
dorf/Württ.) see page 41, "Anginasin", page
260 "Diaporin", page 903
"Struma-Balsam.ISN".**

(73) Titulaire : **BESNOUIN, Didier**
**La Cochère**
**F-61310 Exmes (FR)**

(72) Inventeur : **BESNOUIN, Didier**
**La Cochère**
**F-61310 Exmes (FR)**

(74) Mandataire : **Casalonga, Axel et al**
**BUREAU D.A. CASALONGA - JOSSE**
**Morassistrasse 8**
**W-8000 München 5 (DE)**

EP 0 378 549 B1

## Description

La présente invention est relative à une nouvelle composition pharmaceutique destinée notamment à la prévention et au traitement des maladies bactériennes ou virales non spécifiques.

Classiquement, depuis PASTEUR, on distingue deux grands types de prévention des maladies contagieuses d'origine bactérienne ou virale :

LA PROPHYLAXIE SANITAIRE qui tente de protéger les sujets sains en les isolant des malades, voire en abattant les animaux malades, et en tuant les microbes dans les différentes sources de contagion possibles par tous les procédés de désinfection envisageables.

La mise en oeuvre de ce type de prophylaxie est particulièrement aléatoire car il faut se méfier sans cesse de toutes les possibilités de contagion : insectes, vecteurs animés ou inanimés, moyens de transports, possibilité de porteurs chroniques excrétants ou de futurs malades en période d'incubation, etc.

LA PROPHYLAXIE MEDICALE consiste à protéger les individus sains vis-à-vis d'une éventuelle contagion. A part l'antibiothérapie et la chimioprévention en milieu contaminé, cela consiste surtout en la vaccination à partir de souches bactériennes ou virales inactivées ou atténuées artificiellement en laboratoire. Les vaccinations systématiques des cheptels ont montré leur grande efficacité dans des maladies équines comme la grippe, le tétanos, la rage ou encore le botulisme et le charbon.

Par contre, des maladies virales dues à des virus faiblement immunigènes parce qu'entourés d'une membrane cytoplasmique dérivée de l'hote infesté, sont particulièrement difficiles à vacciner; c'est le cas de la Rhinopneumonie Virale, de l'Artérite Virale et d'autres viroses dues à des virus enveloppés.

Le demandeur a découvert maintenant une nouvelle composition permettant d'agir diretement sur les agents de contagion, bactéries ou virus pour stimuler in situ l'organisme pour se débarrasser des agents de contagion en augmentant les défenses naturelles de l'organisme et en stimulant la réponse immunitaire, soit locale, soit sérique.

La composition conforme à l'invention permet essentiellement une prophylaxie de décontamination avec autovaccination.

La composition conforme à l'invention empêche notamment le développement des maladies contagieuses après contamination d'un sujet sain et permet de développer une immunité soit locale, soit générale, contre les germes de contamination, empêche l'animal atteint de transmettre sa maladie et permet de traiter les sujets cliniquement malades dans des formes locales ou éventuellement systémiques.

L'invention a donc pour objet une nouvelle composition pharmaceutique destinée à la prévention et au traitement des maladies d'origine bactérienne ou virale.

D'autres objets de l'invention apparaîtront à la lecture de la description et des exemple qui suivent.

La composition pharmaceutique conforme à l'invention est essentiellement caractérisée par le fait qu'elle contient dans un milieu alcoolique physiologiquement acceptable et antiseptique au moins un agent à effet phlogistique choisi parmi la poudre de Cantharide, Causticum d'Hahneman et/ou l'iode, à des doses homéopathiques, au moins un agent ayant un effet de stimulation des membranes cytoplasmiques eucaryotes choisi parmi les oligo-éléments et/ou le phosphore, à des doses homéopathiques et au moins un agent ayant un effet de tannage et d'inhibition des corés viraux et des membranes bactériennes constitué par de phénol.

La composition conforme à l'invention selon une forme de réalisation préférée peut contenir également des substances actives ayant un effet de congestion des vaisseaux sanguins, de mobilisation leucocytaire, de fluidification des sécrétions et d'inhibition des suppurations choisies plus particulièrement parmi l'acide silicilique, l'arnica de montagne, l'Hepar Sulfort utilisés à des doses homéopathiques.

Cette composition peut également contenir dans une autre forme de réalisation préférée, des agents à effet anti-inflammatoire tels que l'Indigo sauvage, la Bryone.

Les compositions conformes à l'invention peuvent également contenir en plus des agents cités ci-dessus, de l'acide nitrique, du carbonate de calcium, en particulier des huîtres, de l'Echinée d'Amérique du Nord, de l'eau de seiche, la teinture de feuilles de Thuya, l'anémone pulsatile et du fenouil d'eau.

L'alcool préféré est l'alcool éthylique. Les constituants conformes à l'invention sont essentiellement présents dans des proportions suivantes :

| | |
|---|---|
| Alcool éthylique | 20 à 50 % |
| Phénol | 1 à 3 % |

Les autres composants sont utilisés dans des doses homéopathiques exprimées en unité hahnimaniennes (CH).

2

| | | | |
|---|---|---|---|
| Iode | 10 à 100 | CH |
| Acide nitrique | 5 à 40 | CH |
| Poudre de Cantharide | 5 à 60 | CH |
| Causticum d'Hahneman | 20 à 80 | CH |
| Carbonate de calcium | 20 à 60 | CH |
| Oligo-éléments | 10 à 80 | ppm |
| Echinée d'Amérique du Nord | 8 à 20 | ppm |
| Phosphore | 6 à 15 | CH |
| Eau de seiche | 20 à 60 | CH |
| Teinture de feuilles de Thuya | 25 à 60 | CH |
| Acide silicilique | 20 à 60 | CH |
| Arnica de Montagne | 8 à 20 | CH |
| Anémone pulsatile | 20 à 60 | CH |
| Bryone | 8 à 20 | CH |
| Fenouil d'eau | 8 à 20 | CH |
| Hepar Sulfur | 20 à 80 | CH |
| Indigo sauvage | 5 à 35 | CH |

La composition conforme à l'invention peut contenir en plus des substances actives indiquées ci-dessus, des excipients classiques choisis suivant la forme utilisable.

Cette composition peut être conditionnée en particulier dans des bombes aérosols sous protoxyde d'azote pour des pulvérisations aériennes.

Il est également possible de la présenter sous forme de pastilles ou gomme à mâcher pour la protection buccale, d'oblets gynécologiques pour les femelles animales ou les femmes, de crèmes pour la protection des mâles, de suppositoires pour les contaminations anales.

Sans que cette explication soit limitative, la composition conforme à l'invention qui associe médecine allopathique et médecine homéopathique agit directement sur les agents de contagion et stimule les défenses naturelles de l'organisme.

Dans un premier temps, il y a inhibition des bactéries ou virus puis marquage de ceux-ci par un procédé de tannage par le phénol, permettant de préparer in situ des particules virales ou bactériennes vaccinales.

Ces particules en contact avec une muqueuse dont les défenses immunitaires auront été dopées par l'hyperhémie, l'augmentation de l'activité secrétoire et la mobilisation leucocytaire développent une immunité locale de surface, ayant un effet de vaccination vis-à-vis d'une nouvelle infection.

La composition conforme à l'invention par son effet phlogistique, permet également de débusquer les virus enveloppés dans une membrane cytoplasmique dérivée de l'hote tels que l'herpès virus (ADN) ou les togavirus (ARN).

Le demandeur a constaté notamment qu'il était possible d'obtenir un effet de rappel comme dans toutes les vaccinations, grâce à l'application par pulvérisation de la composition, lors de nouveaux contacts contaminants, ce qui a été constaté en particulier pour l'herpès génital du cheval dû à Equine Herpès Virus III.

Cette composition est particulièrement efficace dans le traitement des infections localisées telles que les rhinite, trachéite, sinusite, vaginite, balanoposthites.

La composition s'est révélée particulièrement efficace pour le traitement des maladies respiratoires ou les maladies génitale du cheval et notamment la rhinopneumonie virale équine, l'artérite virale équine, l'herpès génital du cheval.

Il va de soi que l'effet constaté permet également d'appliquer la composition au traitement ou à la prévention de maladies bactériennes ou virales des autres mammifères, y compris l'homme.

L'exemple suivant est destiné à illustrer une réalisation préférée de l'invention.

La composition suivante a été préparée.

## FORMULE D'EQUIBION

| | |
|---|---|
| Acide silicilique | 30 CH |
| Acide nitrique | 12 CH |
| Anémone pulsatile | 30 CH |
| Arnica de montagne | 12 CH |
| Indigo sauvage | 12 CH |
| Bryone | 12 CH |
| Poudre de Cantharide | 12 CH |
| Causticum d'Hahneman | 30 CH |
| Carbonate de chaux (huitre) | 30 CH |
| Echinée d'Amérique du Nord | 12 CH |
| Encre de seiche | 30 CH |
| Fenouil d'eau | 12 CH |
| Hepar sulfur | 30 CH |
| Iode | 30 CH |
| Phosphore blanc | 12 CH |

| | |
|---|---|
| Teinture des feuilles de Thuya | 30 CH |
| Phénol | 2 % |
| Alcool 47,5° | 400 $CM^3$ |
| Eau | 440 $CM^3$ |

Cette composition s'est révélée particulièrement efficace dans le traitement d'un herpès génital du cheval qui est une maladie virale chronique due essentiellement à l'Equine Herpès Virus III.

La composition décrite ci-dessus a été diluée à la de la solution puis mise sous protoxyde d'azote. Cette composition a été appliquée sur les muqueuses génitales, soit 20 secondes de pulvérisation après la saillie.

Par ailleurs, on peut observer également un effet bactériostatique de la composition sur les bactéries suivantes.

Escherichia Coli
Listeria Monocytogènes
Proteus Mirabilis
Staphylococcus Aureus
Staphylococcus bovin. Cette composition ne présente pas d'effet toxique, par exemple l'admistration d'un litre de la solution à la sonde stomacale n'a pas provoqué de trouble majeur chez l'animal.

**Revendications**

1. Composition pharmaceutique, caractérisée par le fait qu'elle contient dans un milieu alcoolique physiologiquement acceptable et antiseptique au moins un agent à effet phlogistique choisi parmi la poudre de Cantharide, Causticum d'Hahneman et/ou l'iode, à des doses homéopathiques, au moins un agent ayant un effet de stimulation des membranes cytoplasmiques eucaryotes choisi parmi les oligo-éléments et/ou le phosphore, à des doses homéopathiques et au moins un agent ayant un effet de tannage et d'inhibition des corés viraux et des membranes bactériennes constitué par du phénol.

4

2. Composition selon la revendication 1, caractérisée par le fait que l'alcool est constitué par de l'alcool éthylique présent dans des proportions de 20 à 50% et du phénol présent dans des proportions comprises entre 1 et 3%.

3. Composition selon l'une quelconque des revendications 1 ou 2, caractérisée par le fait que la composition contient également des substances actives ayant un effet de congestion des vaisseaux sanguins, de mobilisation leucocitaire, de fluidification des sécrétions et d'inhibition des suppurations choisi parmi l'acide silicilique, l'Arnica de montagne, l'Hepar Sulfur, utilisées dans des doses homéopathiques.

4. Composition selon l'une quelconque des revendications 1 à 3, caractérisée par le fait que la composition contient également des agents à effet anti-inflammatoire choisis parmi l'Indigo sauvage, la Bryone, présents dans des doses homéopathiques.

5. Composition selon l'une quelconque des revendications 1 à 4, caractérisée par le fait que la composition contient également de l'acide nitrique, du carbonate de calcium dérivé des huîtres, de l'Echinée d'Amérique du Nord, de l'eau de seiche, de la teinture de feuilles de thuya, de l'anémone pulsatile et du fenouil d'eau, utilisés dans des doses homéopathiques.

6. Composition selon l'une quelconque des revendications 1 à 5, caractérisée par le fait que les agents présents à des doses homéopathiques sont utilisés dans des proportions suivantes :

| | | |
|---|---|---|
| Iode | 10 à 100 | CH |
| Acide nitrique | 5 à 40 | CH |
| Poudre de Cantharide | 5 à 60 | CH |
| Causticum d'Hahneman | 20 à 80 | CH |
| Carbonate de calcium | 20 à 60 | CH |
| Oligo-éléments | 10 à 80 | ppm |
| Echinée d'Amérique du Nord | 8 à 20 | ppm |
| Phosphore | 6 à 15 | CH |
| Eau de seiche | 20 à 60 | CH |
| Teinture de feuilles de Thuya | 25 à 60 | CH |
| Acide silicilique | 20 à 60 | CH |
| Arnica de Montagne | 8 à 20 | CH |
| Anémone pulsatile | 20 à 60 | CH |
| Bryone | 8 à 20 | CH |
| Fenouil d'eau | 8 à 20 | CH |
| Hepar Sulfur | 20 à 80 | CH |
| Indigo sauvage | 5 à 35 | CH |

7. Composition selon l'une quelconque des revendications 1 à 6, caractérisée par le fait que la composition est conditionnée dans des bombes aérosols sous protoxyde d'azote pour des pulvérisations aériennes.

8. Composition selon l'une quelconque des revendications 1 à 6, caractérisée par le fait que la composition se présente sous forme d'oblets gynécologiques, de crèmes, de suppositoires ou de pastilles.

9. Utilisation de la composition telle que définie dans l'une quelconque des revendications 1 à 8, pour la préparation d'un médicament destiné au traitement des maladies respiratoires ou des maladies génitales d'origine virale ou bactérienne transmises par voie aérienne ou génitale chez les mammifères y compris l'homme.

10. Utilisation selon la revendication 9, caractérisée par le fait que le médicament est destiné au traitement de l'herpès génital du cheval, des rhinopneumonies virales équines, de l'artérite virale équine.

**Patentansprüche**

1. Arzneimittelzusammensetzung, dadurch gekennzeichnet, daß sie im physiologischen und antiseptischen Milieu wenigstens einen Wirkstoff mit phlogistischer Wirksamkeit, ausgewählt unter Kantharidenpulver, Causticum Hahnemann und/oder Jod in homöopathischen Dosen, wenigstens einen Wirkstoff mit einer stimulierenden Wirkung auf die eukaryotischen Zytoplasmamembrane, ausgewählt unter den Spurenelementen und/oder Phosphor in homöopathischen Dosen, und wenigstens einen Wirkstoff mit Gerb- und Hemmwirkung auf Virenkerne und aus Phenol bestehende Bakterienmembrane enthält.

2. Zusammensetzung gemäß Anspruch 1, dadurch gekennzeichnet, daß der Alkohol Ethylalkohol (Ethanol) ist, der im Verhältnis 20 bis 50 % enthalten ist, und Phenol im Verhältnis 1 bis 3 % enthalten ist.

3. Zusammensetzung gemäß einem beliebigen der Ansprüche 1 oder 2, dadurch gekennzeichnet, daß die Zusammensetzung ferner Wirkstoffe enthält, die Hyperämie in den Blutgefäßen, Mobilisierung der Leukozyten, Verflüssigung der Sekrete und Inhibition der Eiterabsonderung bewirken, ausgewählt unter Silicilsäure, Arnica montana, Hepar sulfuris in homöopathischen Dosen.

4. Zusammensetzung gemäß einem beliebigen der Ansprüche 1 bis 3, dadurch gekennzeichnet, daß die Zusammensetzung ferner entzündungshemmende Wirkstoffe enthält, ausgewählt unter wildem Indigo, Bryone in homöopathischen Dosen.

5. Zusammensetzung gemäß einem beliebigen der Ansprüche 1 bis 4, dadurch gekennzeichnet, daß die Zusammensetzung auch Salpetersäure, aus Austern gewonnenes Calciumcarbonat, Nordamerikanischen Seeigel, Tintenfischtinte, Thujenblättertinktur, Anemone pulsatilla und Wasserfenchel in homöopathischen Dosen enthält.

6. Zusammensetzung gemäß einem beliebigen der Ansprüche 1 bis 5, dadurch gekennzeichnet, daß die in homöopathischen Dosen eingesetzten Wirkstoffe in den folgenden Verhältnissen gebraucht werden:

| | | | |
|---|---|---|---|
| Jod | 10 | – | 100 CH |
| Salpetersäure | 5 | – | 40 CH |
| Kantharidenpulver | 5 | – | 60 CH |
| Causticum Hahnemann | 20 | – | 80 CH |
| Calciumcarbonat | 20 | – | 60 CH |
| Spurenelemente | 10 | – | 80 ppm |
| Nordamerikanischer Seeigel | 8 | – | 20 ppm |
| Phosphor | 6 | – | 15 CH |
| Tintenfischtinte | 20 | – | 60 CH |
| Thujenblättertinktur | 25 | – | 60 CH |
| Silicilsäure | 20 | – | 60 CH |
| Arnica montana | 8 | – | 20 CH |
| Anemone pulsatilla | 20 | – | 60 CH |
| Bryone | 8 | – | 20 CH |
| Wasserfenchel | 8 | – | 20 CH |
| Hepar Sulfuris | 20 | – | 80 CH |
| Wilder Indigo | 3 | – | 35 CH |

7. Zusammensetzung gemäß einem beliebigen der Ansprüche 1 bis 6, dadurch gekennzeichnet, daß die Zusammensetzung in Aerosolbomben unter Stickstoffoxydul für die Luftzerstäubung konditioniert wird.

8. Zusammensetzung gemäß einem beliebigen der Ansprüche 1 bis 5, dadurch gekennzeichnet, daß die Zusammensetzung in der Form von gynäkologischen Pessaren, Cremes, Zäpfchen oder Pastillen dargestellt wird.

9. Anwendung der Zusammensetzung gemäß einem beliebigen der Ansprüche 1 bis 8 zur Zubereitung eines Heilmittels für die Behandlung der Erkrankungen der Atemwege oder der Genitalien viralen oder bakteriellen Ursprungs auf dem Luftweg oder auf genitalem Weg bei Säugetieren, einschließlich des Menschen.

10. Anwendung gemäß Anspruch 9, dadurch gekennzeichnet, daß das Heilmittel zur Behandlung des Gentialherpes beim Pferd, der viralen Rhinopneumonien beim Pferd, der viralen Arterienentzündung beim Pferd eingesetzt wird.

**Claims**

1. Pharmaceutical composition, characterized in that is contains, in a physiologically acceptable and antiseptic alcoholic medium, at least one agent having an inflammatory effect, chosen from cantharides powder, Hahnemann's Causticum and/or iodine, at homoeopathic doses, at least one agent having a stimulatory effect on eukaryotic cytoplasmic membranes, chosen from trace elements and/or phosphorus, at homoeopathic doses, and at least one agent having a tanning and inhibitory effect on viral cores and bacterial membranes, consisting of phenol.

2. Composition according to Claim 1, characterized in that the alcohol consists of ethyl alcohol, present in proportions of 20 to 50%, and of phenol present in proportions between 1 and 3%.

3. Composition according to either of Claims 1 and 2, characterized in that the composition also contains active substances having an effect of congestion of blood vessels, of leukocyte mobilization, of fluidification of secretions and of inhibition of suppurations, chosen from silicic acid, Arnicas montana and Hepar Sulphuris, used at homoeopathic doses.

4. Composition according to any one of Claims 1 to 3, characterized in that the composition also contains agents having an anti-inflammatory effect, chosen from wild indigo and Bryonia, present in homoeopathic doses.

5. Composition according to any one of Claims 1 to 4, characterized in that the composition also contains nitric acid, calcium carbonate derives from oysters North American sea-urchin, cuttlefish water, tincture of Thuja leaves, Pulsatilla and water fennel, used in homoeopathic doses.

6. Composition according to any one of Claims 1 to 5, characterized in that the agents present at homoeopathic doses are used in the following proportions:

| | | | |
|---|---|---|---|
| Iodine | 10 | to | 100 CH |
| Nitric acid | 5 | to | 40 CH |
| Cantharides powder | 5 | to | 60 CH |
| Hahnemann's Causticum | 20 | to | 30 CH |
| Calcium carbonate | 20 | to | 60 CH |
| Trace elements | 10 | to | 80 ppm |
| North American sea-urchin | 8 | to | 20 ppm |
| Phosphorus | 6 | to | 15 CH |
| Cuttlefish water | 20 | to | 60 CH |
| Tincture of Thuja leaves | 25 | to | 60 CH |
| Silicic acid | 20 | to | 60 CH |
| Arnica montana | 8 | to | 20 CH |
| Pulsatilla | 20 | to | 60 CH |
| Bryonia | 8 | to | 20 CH |
| Water fennel | 8 | to | 20 CH |
| Hepar Sulphuris | 20 | to | 80 CH |
| Wild indigo | 5 | to | 35 CH |

7. Composition according to any one of Claims 1 to 6, characterized in that the composition is packaged in areosol cans under nitrous oxide for aerial spraying.

8. Composition according to any one of Claims 1 to 6, characterized in that the composition takes the form of gynaecological pessaries, creams, suppositories or pastilles.

9. Use of the composition as defined in any one of Claims 1 to 8, for the preparation of a medicinal product intended for the treatment of respiratory diseases or genital diseases of viral or bacterial origin, transmitted aerially or genitally in mammals, including man.

10. Use according to Claim 9, characterized in that the medicinal product is intended for the treatment of genital herpes of horses, equine viral rhinopneumonitis and equine viral arteritis.